# EUROPEAN PATENT APPLICATION

(11) **EP 4 703 713 A1**
(43) Date of publication of application: **04.03.2026**
(21) Application number: 25198494.4
(22) Date of filing: 27.08.2025
(51) Int. Cl.: G01N 23/04, G01T 1/17, G01T 1/40

(54) **TEMPERATURE DETECTION AND COMPENSATION IN A MEDICAL IMAGING SYSTEM**

(30) Priority: 30.08.2024 US 202418821599
(71) Applicant: Avago Technologies International Sales Pte. Limited, Singapore 768923 (SG)
(72) Inventor: Enne, Reinhard, 3202 Hofstetten-Gruenau (AT); Gaberl, Wolfgang, 1220 Vienna (AT); Davidovic, Milos, 1110 Vienna (AT)
(74) Representative: Dilg, Haeusler, Schindelmann Patentanwaltsgesellschaft mbH

(57) **Abstract**

An example apparatus for temperature compensation in an imaging system (100) is described. The apparatus includes an analog front end (302) configured to receive a signal from a detector (104) of the imaging system (100), the analog front end (302) being integrated in an integrated circuit (IC) (106) electrically and thermally coupled to the detector (104); a low-pass filter (306) coupled to an output of the analog front end (302) and configured to output a first signal; a first circuit configured to output a second signal based on power consumed by the IC (106); and a second circuit configured to receive the first and second signals and output a third signal in response to the first and second signals.

## Description

### BACKGROUND

In advanced Computed Tomography (CT) and Positron Emission Tomography (PET) scanner detection architectures, there is a growing emphasis on concern for temperature dependence as these systems have evolved into more sophisticated versions. The temperature effects can affect detector performance and signal quality of the detector output. Consequently, measuring temperature, such as relative temperature variation, has become a predominant design consideration for state-of-the-art CT and PET scanner systems.

An important challenge arises from the fact that detectors are typically not equipped with temperature-measuring devices of desired performances. As a result, the measurement of their temperature can only be achieved indirectly by monitoring spots in very close proximity to the detectors, where a dedicated temperature sensor may be attached. This does not only take an extra discrete component that has to be designed into the system, but also does not give exact data about the heat dissipation at the detector itself.

An additional challenge involves responding swiftly to temperature regulation changes. Current state-of-the-art methods can employ air-to-air or water-to-air cooling techniques, utilizing heat sinks attached to thermal dissipaters. However, this approach can introduce an extra time constant that inherently restricts temperature stability. In other words, in the actual state-of-the-art PET and CT systems there can be a certain temperature oscillation range under which the system cannot go. Consequently, this limitation can impact the overall performance of both PET and CT scanners, potentially affecting their operational efficiency.

### SUMMARY

In an embodiment, an apparatus for temperature compensation in an imaging system is described. The apparatus includes an analog front end configured to receive a signal from a detector of the imaging system, the analog front end being integrated in an integrated circuit (IC) electrically and thermally coupled to the detector. The apparatus includes a low-pass filter coupled to an output of the analog front end and configured to output a first signal. The apparatus includes a first circuit configured to output a second signal based on power consumed by the IC. The apparatus includes a second circuit configured receive the first and second signals and output a third signal in response to the first and second signals.

In an embodiment, an apparatus for temperature compensation in an imaging system is described. The apparatus includes a detector and an integrated circuit (IC) electrically coupled to the detector, the IC further thermally coupled to the detector through a thermal reservoir of the imaging system. The IC includes an analog front end configured to receive a signal from the detector, a low-pass filter coupled to an output of the analog front end and configured to output a first signal, and a first circuit configured to output a second signal based on power consumed by the IC. The apparatus includes a second circuit configured receive the first and second signals and output a third signal in response to the first and second signals.

In an embodiment, a method of temperature compensation in an imaging system is described. The method includes receiving a signal at an analog front end from a detector of the imaging system, the analog front end being integrated in an integrated circuit (IC) electrically and thermally coupled to the detector. The method includes low-pass filtering an output of the analog front end to output a first signal. The method includes outputting a second signal based on power consumed by the IC. The method includes outputting a third signal in response to the first and second signals. The method includes dissipating power in a power dissipation element from a power supply of the IC in response to the third signal.

### BRIEF DESCRIPTION OF THE DRAWINGS

Fig. 1 is a block diagram depicting an imaging system that can be used in medical applications according to embodiments.
Fig. 2 is a schematic diagram depicting an electrical and thermal model of the imaging system of Fig. 1.
Fig. 3 is a block diagram depicting an IC coupled to a detector according to embodiments.
Fig. 4 is a block diagram depicting an IC coupled to a detector according to further embodiments.
Fig. 5 is a flow diagram depicting a method of temperature compensation in a sensor front end of an imaging system according to embodiments.

### DETAILED DESCRIPTION

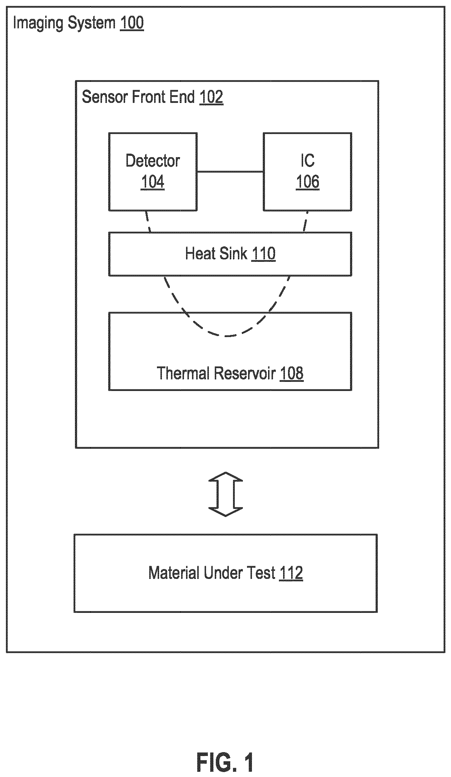

Fig. 1 is a block diagram depicting an imaging system 100 that can be used in medical applications according to embodiments. In embodiments, imaging system 100 can be a positron emission tomography (PET) imaging system. A PET imaging system may be a device that uses radioactive substances (referred to as radiotracers) to visualize and measure changes in physiological activities. In other embodiments, imaging system 100 can be a computer tomography (CT) scan imaging system. A CT scan imaging system may be a device that uses a rotating X-ray tube and detectors to measure X-ray attenuations by different body tissues. Both PET and CT scan imaging systems are well-known in the art. In embodiments, imaging system 100 includes an integrated circuit (IC) 106 that includes circuits, software/firmware, or both for implementing embodiments of temperature detection and compensation described herein. An IC may be an electronic device having components (e.g., transistors, resistors, capacitors) etched onto semiconductor material.

Imaging system 100 can include a sensor front end 102 that interacts with a material under test 112. Material under test 112 may be any type of material capable of being imaged by imaging system 100 (e.g., a human body, organic tissue, etc.). Sensor front end 102 may be a subsystem of imaging system 100 that performs detection and processing. While a single sensor front end 102 is shown, in other embodiments, imaging system 100 can include multiple sensor front ends 102. Sensor front end 102 can include a detector 104 and an IC 106. Detector 104 may be any device that can perform detection based on the type of imaging system 100 (e.g., a detector in a PET imaging system, a detector in a CAT scan system, etc.). Detector 104 can measure a quantity of interest and generate electrical signals. The electrical signals can convey the measurements made by detector 104. Detector 104 can be electrically coupled to IC 106 and can send the electrical signals to IC 106 for processing. In addition, detector 104 can be thermally coupled to IC 106 through a thermal reservoir 108. Thermal reservoir 108 can be any medium through which heat can be conducted. For example, thermal reservoir 108 can be air or a liquid (e.g., water). In embodiments, detector 104 and IC 106 can be thermally coupled to a heat sink 110. Heat sink 110 can be any heat exchanger that transfers heat generated by detector 104 and IC 106 to thermal reservoir 108.

Temperature effects can have an influence on performance of detector 104, significantly affecting the electrical signal quality of the detector output, and therefore limiting the sensitivity of imaging system 100. Thus, measuring temperature, such as relative temperature variation, can be a predominant design consideration for imaging systems 100, such as PET and CT scan imaging systems.

Detector 104 can be sensitive to the physical quantities that are of interest. Detector 104 can supply electrical signals conveying the physical quantities of interest to IC 106, which processes the electrical signals. When the detector's response is of an electrically accessible nature, such as voltage, current, capacitance, resistance, inductance, or frequency, IC 106 can be electrically coupled to detector 104 to sense its output. IC 106 can be a Complementary Metal-Oxide-Semiconductor (CMOS) IC, Bipolar CMOS (BiCMOS) IC, Indium Gallium Arsenide (InGaAs) IC, Indium Phosphide (InP) IC, or the like.

In some embodiments, intermediate stages may be introduced. For example, in a PET scanner system, detector 104 can be a scintillator that converts radiation into light. Sensing this light directly can be challenging due to the system setup constraints, leading to the consideration of optical detectors such as silicon photomultipliers (SiPM) or similar alternatives. An intermediate stage, for example, can convert optical signals into electrical signals for processing by IC 106. However, embodiments described herein function the same or similarly regardless of the presence or absence of such intermediate stages.

The different implementations of imaging system 100 can face a shared challenge: the impact of temperature effects on performance leading to a drift in the detector and/or front-end operating point, which in turn reduces the system sensitivity. If the temperature is not stabilized or the effects are not sufficiently compensated in the post-processing by IC 106, it can lead to inaccurate measurement results. While it can be feasible to compensate for this error to some extent during post-processing, a residual uncertainty arising from these temperature effects remains.

The challenges can be understood with respect to Fig. 1. As shown, sensor front end 102 includes thermal reservoir 108, which can exhibit an imperfect thermal connection to heat sink 110, a temperature-forcing system, or the ambient environment (e.g., heat sink 110 is shown in the example). In other embodiments, detector 104 and IC 106 may not share the same thermal reservoir.

During the operation, heat can be generated within detector 104, IC 106, or both, which is subsequently transferred to heat sink 110 (or the ambient environment or other temperature-forcing system). Due to imperfect thermal conduction from detector 104 and IC 106 to heat sink 110, a perceivable temperature gradient and, consequently, a specific temperature difference can occur. As all components possess heat capacitance, the temperatures undergo changes with a certain delay.

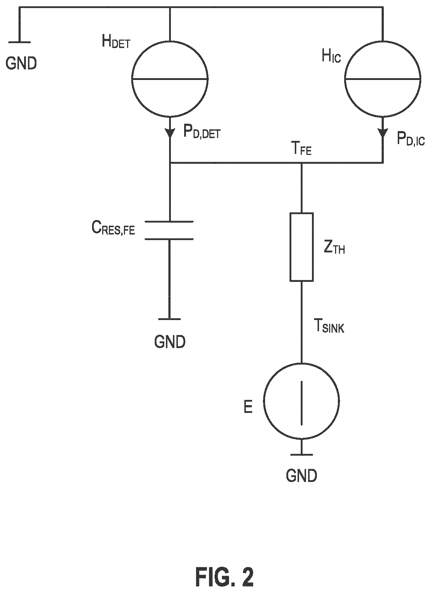

Fig. 2 is a schematic diagram depicting an electrical and thermal model of imaging system 100. As shown in Fig. 2, H_{DET} and H_{IC} can represent heat generation by detector 104 and IC 106, respectively. A capacitance C_{RES,FE} can be a thermal capacitance of thermal reservoir 108 of sensor front end 102. An impedance Z_{TH} can be a thermal resistance between a parallel combination of detector 104 and IC 106, and heat sink 110 (shown as E). The symbol GND in Fig. 2 denotes electrical ground.

If the detector activity and the associated heat generation are very dynamic in nature, the precision of temperature forcing or error compensation can suffer from the fact that the speed of temperature measuring, forcing, or both can be limited by the thermal time constants, as can be perceived from Figure 2. The detector activity may not have a direct connection to the physical quantities of interest, but the detector activity can have a connection to its dissipated power (denoted by P_{D,DET}). Likewise, IC 106 can have a connection to its dissipated power (denoted P_{D,IC}). Embodiments described herein can take advantage of the dissipated power in sensor front end 102. The temperature variation measurement can serve two purposes: first, for temperature stabilization by a cooling/heating device during operation, and second, for error compensation during post-processing.

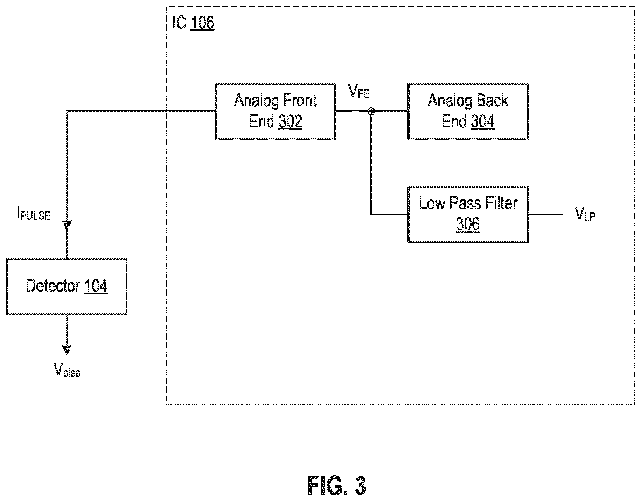

Fig. 3 is a block diagram depicting IC 106 coupled to detector 104 according to embodiments. IC 106 can include an analog front end 302, an analog back end 304, and a low pass filter 306. Analog front end 302 may be circuits that receive electrical signal(s) from detector 104. In the embodiment shown, analog front end 302 receives a current signal (I_{PULSE}) (e.g., supplies a current I_{PULSE} to detector 104). Detector 104 can receive a voltage for operation (shown as V_{bias}). Analog front end 302 can generate a voltage signal (V_{FE}) as output in response to receiving the electrical signal. Analog back end 304 may be circuits that process the output of analog front end (e.g., V_{FE}). For example, analog back end 304 can perform the post-processing operations for imaging system 100. Low pass filter 306 may be a circuit that passes electrical signals with a frequency lower than a selected cut-off frequency and attenuates electrical signals with frequencies higher than the selected cut-off frequency. An input of low pass filter 306 receives the output of analog front end 302 (e.g., V_{FE}). An output of low pass filter 306 supplies an electrical signal (e.g., a voltage signal V_{LP}).

Embodiments described herein provide an approach to monitor variations in detector temperature indirectly through tracking of the detector power dissipation by IC 106. By measuring the energy consumption at all of the consumers, temperature fluctuations can be accurately monitored. In a typical PET or CT scanner, for example, the dominant power consumers can be detector 104 and IC 106. Moreover, the electrical speed at which the measurements can be carried out can outpace any temperature time constant in the system. As such, a temperature variation, which can depend on the dissipated power, can be predicted. Detectors and sensors in imaging systems (e.g., detector 104), such as PET and CAT systems, may not have integrated sensors for measuring IC temperature directly or indirectly using power dissipation. Embodiments described herein measure detector power dissipation using IC 106 and model the temperature variation based on such measured power dissipation.

In operation, analog front end 302 can be electrically coupled to detector 104 and can sense analog activity from detector 104. For example, analog front end 302 can sense detector activity by measuring the current injected or pulled to/from analog front end 302 (e.g., I_{PULSE}), depending on the specific detector type. The current direction is not a subject of the embodiments. Given that detector 104 can be biased with a known bias voltage, it can be deduced that the power dissipation of detector 104 can be calculable by multiplying the known bias voltage (V_{bias}) by the sensed current (I_{PULSE}). Conversely, if detector 104 has a constant current, varying the voltage that is sensed by analog front end 302, the power dissipation can be again calculated. Embodiments are not directed the detector itself, but rather methods for utilizing the signal information present within the system. This process can include extracting signal information and calculating the power dissipation of detector 104 within analog front end 302, thus facilitating the development of a model to predict temperature behavior.

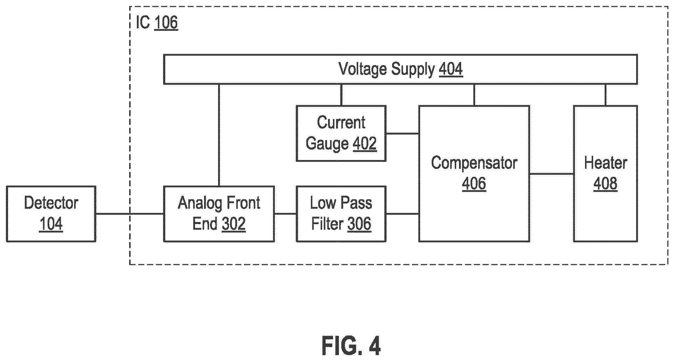

In operation, low pass filter 306 may be configured to extract power dissipation within a specific bandwidth (e.g., by setting the cut-off frequency) that is most relevant to the needs of the system as a whole. The time constant of low pass filter 306 can play a role in this setup - it can be tuned to align with the overall system requirements. Depending on the performance goals of the system, this time constant associated with low pass filter 306 can be in harmony with the operational speed of the temperature control component (embodiments described below in Fig. 4). The speed at which temperature control operates can be faster than the intrinsic thermal time constant (discussed further below).

In embodiments, the time constant of low pass filter 306 can be adjusted within a broad range, which can provide an opportunity to react faster than the real temperature variation can happen in the system. With regards to that, embodiments described below can have a temperature compensation component acting fast enough to compensate for any temperature variation before a potential variation happens. This can be important considering that the detector and the IC can be not only electrically, but also thermally closely coupled.

Fig. 4 is a block diagram depicting IC 106 coupled to detector 104 according to further embodiments. Analog front end 302 and low pass filter 306 are discussed above with respect to Fig. 3. Note that analog back end 304 is omitted from Fig. 4 for clarity (shown in Fig. 3). IC 106 can include a voltage supply 404. Voltage supply 404 may be a circuit that supplies one or more voltages for circuits of IC 106. Circuits that receive voltage from voltage supply 404 can include, for example analog front end 302, current gauge 402, compensator 406, and heater 408. Current gauge 402 may be a circuit that measures current consumed by circuitry of IC 106 from voltage supply 404 (e.g., analog front end 302, compensator 406, heater 408, analog back end 304). Inputs of compensator 406 can be coupled to outputs of current gauge 402 and low pass filter 306. An output of compensator 406 can be coupled to an input of heater 408. Compensator 406 can be a circuit that performs a comparison using outputs of current gauge 402 and low pass filter 306 and generates a resulting output signal. For example, compensator 406 can apply a gain to the outputs of current gauge 402 and low pass filter 406 and compare those outputs against a reference. The result of the comparison can be supplied as output of compensator 406. Heater 408 may be a circuit that dissipates power depending on a control signal. In the embodiment, the output of compensator 406 can supply the control signal for heater 408 in order to control the power dissipated by heater 408. Heater 408 can cause IC 106 to radiate more or less heat depending on the power dissipated by heater 408 under control of compensator 406.

In operation, heater 408 can be a power dissipating element integrated within IC 106 that regulates the temperature of IC 106 and consequently detector 104 or sensor front end 102 as a whole. The function of heater 408 can be to balance the total power consumption of IC 106 such that there is little or ideally no temperature variation at detector 104. The balancing can be performed by dissipating a certain predefined amount of power during an idle status. Compensator 406 can detect power dissipation by detector 104 by monitoring the output of low pass filter 306. Compensator 408 can also detect power dissipation by IC 106 by monitoring the output of current gauge 402. When power dissipation of detector 104, IC 106, or both is occurring, the compensator 406 can reduce power dissipation of heater 408. When power dissipation of detector 104, IC 106, or both abates, the compensator 406 can increase power dissipation of heater 408. In this manner, compensator 406 can adjust power dissipation of heater 408 in response to changes in power dissipation of detector 104, IC 106, or both. The heat generated by IC 106 can thus remain constant or near constant. As such, the heat of detector 104 or sensor front end 102 as a whole can remain constant or near constant.

Embodiments can provide a low time constant on the power dissipation regulation. Heater 408 can be a simple resistor, transistor, diode, inductor, capacitor or any other integrated element available in the integrated technology that can consume enough energy to fulfill the operation described above. The power dissipation can be controlled based on the measured power dissipation from the method described above. The control can be carried out using known techniques, such as proportional (P), proportional integral (PI) or proportional integral derivative (PID) regulation. The temperature regulation system does not have to react to the temperature but on the power dissipation that happens ahead of any temperature change (e.g., temperature change lags power dissipation). This feature can allow prediction of the temperature variation tendency and to act proactively preventing the temperature variation from happening at all (or mitigating the temperature variation). Alternatively, the regulation of the heater's dissipation could be achieved through measured temperature (taken from IC 106 or an external sensor), or a hybrid approach that involves both temperature and the power dissipation of detector 104, IC 106, or both.

In embodiments, heater 408 can be integrated in IC 106. In other embodiments, heater 408 can be implemented external to IC 106 (e.g., as a discrete component in sensor front end 102). In embodiments, the control logic for heater 408 (e.g., compensator 406) can be integrated in IC 106. In other embodiments, the control logic for heater 408 (e.g., compensator 406) can be external to IC 106 (e.g., configured to receive signals from low pass filter 306 and current gauge 402 externally from IC 106).

The embodiments described above include circuitry in IC 106 to measure power dissipation of detector 104. Analog front end 302 can be fabricated using a standard deep-submicron CMOS or BiCMOS process, but is not limited to the silicon only, as GaAs InP or other technologies could be employed as well. In embodiments, analog front end 302 can include a function to sense the current of consumed by detector 104 keeping the voltage constant. Under post-processing by analog back end 304, a shape filtering, bandwidth limitation, DC cancelation or other techniques known in the art can be used to extract the signal information of interest. In this case, analog front end 302 can be a transimpedance amplifier (TIA) circuit.

Depending on the application, this analog domain post-processing may instead be realized in the current domain. In case of current post-processing, analog front end 302 can be a current conveyor (CC) circuit. There are many forms of TIAs and CCs known in the art that can be used in the embodiments.

Regardless of how the low-pass filter branch is incorporated into a Transimpedance Amplifier (TIA), a current conveyor, or any other sensing circuit responsible for detector sensing, the output of this low-pass filtered signal can be transmitted to the control logic for temperature regulation control (e.g., compensator 406). The transmission of this signal can be achieved through various methods, including but not limited to voltage buffers/amplifier, current buffers with or without gain if analog control input is desired. In case of a digital signal request, an Analog-to-Digital Converter (ADC) can be integrated within IC 106, or can be realized externally, outside of IC 106. These methods can ensure the effective and efficient relay of the filtered signal to the control logic for further processing.

In embodiments, IC 106 can incorporate several analog front-ends having each of them connected to a separate detector. In this case, a low pass filter could be integrated with each of the analog front-ends outputting a value proportional to the dissipation change of the corresponding detector. Alternatively, one low-pass filter could receive from several analog front-ends and average out the dissipation of two or more corresponding detectors.

To enhance temperature control, the system described can incorporate a power-dissipating component integrated into IC 106 (e.g., heater 408). Heater 408 can be a current mirror or the like capable of drawing a controllable amount of current from voltage supply 404 and conducting the current to generate heat in IC 106. In embodiments, incorporating multiple dissipating elements within IC 106 can be beneficial, e.g., for ICs designed to connect with multiple detectors. In case of multiple dissipating elements, those dissipating elements can be located in different areas of IC to provide temperature regulation at specific locations in the IC, e.g., the location(s) where detector 104 is thermally coupled to IC 106 (e.g., heat sink locations).

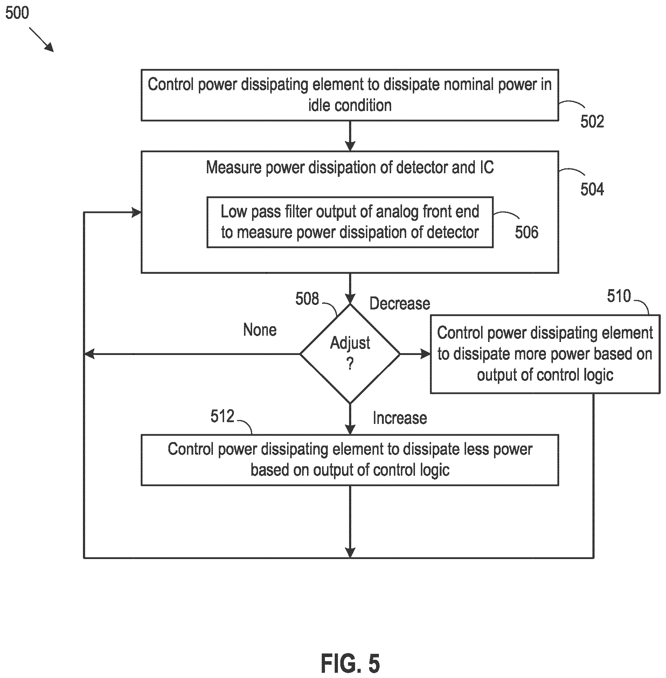

Fig. 5 is a flow diagram depicting a method 500 of temperature compensation in a sensor front end of an imaging system according to embodiments. Method 500 begins at step 502, where compensator 406 controls heater 408 (a power dissipating element) to dissipate nominal power in an idle condition. That is, the power dissipating element dissipates some nominal power such that a nominal temperature condition can be maintained at detector 104. Since IC 106 and detector 104 are thermally coupled, the controlling temperature of IC 106 can control temperature of detector 104. During the idle condition, the temperature of the IC 106 and detector 104 can be at equilibrium as set by the nominal power dissipated by the dissipating element.

At step 504, compensator 406 measures power dissipation of detector 104 and IC 106. For example, at step 506, compensator 406 can receive a low pass filtered output of analog front end 302 in order to measure power dissipation of detector 104. Compensator 406 can receive output of current gauge 402 or similar power detector for detecting power consumed by IC 106. At step 508, compensator 406 determines whether to adjust the power dissipating element. If no adjustment is necessary, method 500 returns to step 504. In case of an increase in power dissipation, method 500 proceeds to step 512. At step 512, either detector 104 or IC 106 or both show an increase in power dissipation. Thus, at step 512, compensator 406 can control the power dissipating element to dissipate less power. This will cause IC to decrease in temperature. Detector 104 may decrease in temperature either by dissipating less power or through thermal coupling with IC 106. In either case, there is little or no temperature gradient between detector 104 and IC 106 due to the adjustment made by compensator 406.

In case of a decrease in power dissipation, method 500 proceeds from step 508 to step 510. At step 510, either detector 104 or IC 106 or both show a decrease in power dissipation. Thus, at step 510, compensator 407 can control the power dissipating element to dissipate more power. This will cause IC to increase in temperature. Detector 104 may increase in temperature either by dissipating more power or through thermal coupling with IC 106. In either case, there is little or no temperature gradient between detector 104 and IC 106 due to the adjustment made by compensator 406.

While some processes and methods having various operations have been described, one or more embodiments also relate to a device or an apparatus for performing these operations. The apparatus may be specially constructed for required purposes, or the apparatus may be a general-purpose computer selectively activated or configured by a computer program stored in the computer. Various general-purpose machines may be used with computer programs written in accordance with the teachings herein, or it may be more convenient to construct a more specialized apparatus to perform the required operations.

One or more embodiments of the present invention may be implemented as one or more computer programs or as one or more computer program modules embodied in computer readable media. The term computer readable medium refers to any data storage device that can store data which can thereafter be input to a computer system. Computer readable media may be based on any existing or subsequently developed technology that embodies computer programs in a manner that enables a computer to read the programs. Examples of computer readable media are hard drives, NAS systems, read-only memory (ROM), RAM, compact disks (CDs), digital versatile disks (DVDs), magnetic tapes, and other optical and non-optical data storage devices. A computer readable medium can also be distributed over a network-coupled computer system so that the computer readable code is stored and executed in a distributed fashion.

As used herein, the phrase "at least one of" preceding a series of items, with the term "and" or "or" to separate any of the items, modifies the list as a whole rather than each member of the list (i.e., each item). The phrase "at least one of" does not require selection of at least one of each item listed; rather, the phrase allows a meaning that includes at least one of any one of the items, and/or at least one of any combination of the items. By way of example, the phrases "at least one of A, B, and C" or "at least one of A, B, or C" each refer to only A, only B, or only C; and/or any combination of A, B, and C. In instances where it is intended that a selection be of "at least one of each of A, B, and C," or alternatively, "at least one of A, at least one of B, and at least one of C," it is expressly described as such.

As used herein, the term "couple" and its derivatives include: (a) electrical and communicative coupling; and (b) do not imply a direct connection, but rather may include intervening elements, unless described as "directly coupled."

Although one or more embodiments of the present invention have been described in some detail for clarity of understanding, certain changes may be made within the scope of the claims. Accordingly, the described embodiments are to be considered as illustrative and not restrictive, and the scope of the claims is not to be limited to details given herein but may be modified within the scope and equivalents of the claims. In the claims, elements and/or steps do not imply any particular order of operation unless explicitly stated in the claims.

Boundaries between components, operations, and data stores are somewhat arbitrary, and particular operations are illustrated in the context of specific illustrative configurations. Other allocations of functionality are envisioned and may fall within the scope of the invention. In general, structures and functionalities presented as separate components in exemplary configurations may be implemented as a combined structure or component. Similarly, structures and functionalities presented as a single component may be implemented as separate components. These and other variations, additions, and improvements may fall within the scope of the appended claims.

## Claims

1. An apparatus for temperature compensation in an imaging system (100), comprising:
an analog front end (302) configured to receive a signal from a detector (104) of the imaging system (100), the analog front end (302) being integrated in an integrated circuit, IC, (106) electrically and thermally coupled to the detector (104);
a low-pass filter (306) coupled to an output of the analog front end (302) and configured to output a first signal;
a first circuit configured to output a second signal based on power consumed by the IC (106); and
a second circuit configured receive the first and second signals and output a third signal in response to the first and second signals.

2. The apparatus of claim 1, further comprising:
a power dissipating element (408), coupled to the second circuit, configured to dissipate power from a power supply of the IC (106) in response to the third signal.

3. The apparatus of claim 2, wherein the power dissipating element (408) is integrated in the IC (106).

4. The apparatus of any of the claims 1 to 3, wherein the second circuit is configured to measure power dissipation of the detector (104) from the first signal and power dissipation of the IC (106) from the second signal.

5. The apparatus of claim 4, wherein the second circuit is configured to increase power dissipated by the power dissipating element (408) in response to a decrease in power dissipation indicated by the first signal, the second signal, or both the first and second signals.

6. The apparatus of claim 4 or 5, wherein the second circuit is configured to decrease power dissipated by the power dissipating element (408) in response to an increase in power dissipation indicated by the first signal, the second signal, or both the first and second signals.

7. The apparatus of any of the claims 1 to 6, comprising at least one of the following features:
wherein the second circuit is configured to control the power dissipating element (408) to dissipate nominal power in an idle condition;
wherein the power dissipating element (408) comprises a plurality of circuits disposed in different locations of the IC (106).

8. An apparatus for temperature compensation in an imaging system (100), comprising:
a detector (104);
an integrated circuit, IC, (106) electrically coupled to the detector (104), the IC (106) further thermally coupled to the detector (104) through a thermal reservoir of the imaging system (100), the IC (106) including:
an analog front end (302) configured to receive a signal from the detector (104);
a low-pass filter (306) coupled to an output of the analog front end (302) and configured to output a first signal;
a first circuit configured to output a second signal based on power consumed by the IC (106);
a second circuit configured receive the first and second signals and output a third signal in response to the first and second signals.

9. The apparatus of claim 8, further comprising:
a power dissipating element (408), coupled to the second circuit, configured to dissipate power from a power supply of the IC (106) in response to the third signal;
in particular wherein the power dissipating element (408) is integrated in the IC (106).

10. The apparatus of claim 8 or 9, comprising at least one of the following features:
wherein the second circuit is integrated in the IC (106);
further comprising a heat sink configured to thermally couple the detector (104) and the IC (106).

11. The apparatus of any of the claims 8 to 10, wherein the second circuit is configured to measure power dissipation of the detector (104) from the first signal and power dissipation of the IC (106) from the second signal.

12. The apparatus of claim 11, comprising at least one of the following features:
wherein the second circuit is configured to increase power dissipated by the power dissipating element (408) in response to a decrease in power dissipation indicated by the first signal, the second signal, or both the first and second signals;
wherein the second circuit is configured to decrease power dissipated by the power dissipating element (408) in response to an increase in power dissipation indicated by the first signal, the second signal, or both the first and second signals.

13. A method of temperature compensation in an imaging system (100), comprising:
receiving a signal at an analog front end (302) from a detector (104) of the imaging system (100), the analog front end (302) being integrated in an integrated circuit, IC, (106) electrically and thermally coupled to the detector (104);
low-pass filtering an output of the analog front end (302) to output a first signal;
outputting a second signal based on power consumed by the IC (106);
outputting a third signal in response to the first and second signals; and
dissipating power in a power dissipation element from a power supply of the IC (106) in response to the third signal.

14. The method of claim 13, wherein the step of outputting the third signal comprises:
measuring power dissipation of the detector (104) from the first signal;
measuring power dissipation of the IC (106) from the second signal; and
generating the third signal in response to the first and second signals.

15. The method of claim 14, comprising at least one of the following features:
further comprising:
increasing power dissipated by the power dissipating element (408) in response to a decrease in power dissipation indicated by the first signal, the second signal, or both the first and second signals;
further comprising:
decreasing power dissipated by the power dissipating element (408) in response to an increase in power dissipation indicated by the first signal, the second signal, or both the first and second signals.
